Europäisches Patentamt

European Patent Office

Office européen des brevets

(11) Publication number: **0 289 219**

**A2**

(12) **EUROPEAN PATENT APPLICATION**

(21) Application number: 88303610.5

(22) Date of filing: 21.04.88

(51) Int. Cl.4: **G01N 33/82** , **C12Q 1/00** , **C12Q 1/32**

(30) Priority: 27.04.87 JP 101740/87

(43) Date of publication of application:
02.11.88 Bulletin 88/44

(84) Designated Contracting States:
CH DE FR GB IT LI NL

(71) Applicant: FUJIREBIO KABUSHIKI KAISHA
also trading as FUJIREBIO INC.
6-7, Shimoochiai 4-chome Shinjuku-ku
Tokyo 161(JP)

(72) Inventor: Ashihara, Yoshihiro
2-402, Fuchudanchi 28-1, Harumicho
1-chome
Fuchu-shi Tokyo(JP)
Inventor: Kasahara, Yasushi
310, 4-4, Nagayama 3-chome
Tama-shi Tokyo(JP)

(74) Representative: Silverman, Warren et al
HASELTINE LAKE & CO. Hazlitt House 28
Southampton Buildings Chancery Lane
London WC2A 1AT(GB)

(54) **Method of measuring biotin.**

(57) A method of measuring biotin comprises contacting avidin or streptoavidin with the biotin to be measured and a biotinyl enzyme, and measuring the biotinyl enzyme activity.

EP 0 289 219 A2

# METHOD OF MEASURING BIOTIN

This invention relates to a method of measuring biotin. More particularly, this invention relates to a method of measuring biotin by allowing a biotinyl enzyme and avidin or streptoavidin to react competitively with biotin in a sample, and measuring the activity of the remaining biotinyl enzyme.

Biotin was found as anti-egg-white-injury factor, and now, it is known as vitamin H. Biotin functions as the coenzyme for principal enzymes in the metabolism of fatty acids, such as in carbonate fixation, carbonate transfer and decarboxylation. It is known that when a person ingests too much egg white, there is a decrease in the amount of available biotin. Recently, metabolic error in newborn babies caused by biotinidase deficiency has been found, and therefore, it is necessary to develop a simple method capable of determining accurately biotin in serum samples.

Known methods of measuring biotin are bioassay, the DACA method and the avidin method and heretofore, the bioassay was widely utilized because of simple operation. However, the bioassay has a problem in the reliability of the results which varies according to for example the microorganism used. Moreover, a reagent for the determination of biotin has been developed (Japanese Patent KOKAI Nos.71359/1986 and 149863/1986). However, when using this reagent, sensitivity is not very high and the analyzing of results is still complicated.

In the meanwhile the present inventors have developed a method of measuring biological ligand by utilizing the reaction between a biotinyl enzyme and avidin or streptoavidin (EPA 84304093.2 (Publication Ser.No. 0132292), Japanese Patent KOKAI No.142466/1984).

An object of the invention is to provide a method capable of measuring biotin accurately in a simple operation.

Another object of the invention is to provide a method of measuring biotin in a high sensitivity.

The present invention provides a method of measuring biotin which enables both objects to be met. The method comprises contacting avidin or streptoavidin with the biotin to be measured and a biotinyl enzyme, and measuring the residual biotinyl enzyme activity.

Biotinyl enzyme is the name given to a number of enzymes having a biotinyl group at their active site, which enzymes include propionyl CoA carboxylase, acetyl CoA carboxylase, pyruvate carboxylase, methylmalonyl CoA carboxylase, methylmalonyl CoA transcarboxylase, and methylcrotonyl CoA carboxylase. The term biotinyl enzyme also includes the biotin-modified enzymes prepared from other enzymes by reacting them with activated biotin. For example, biotin-induced glucose-6-phosphate dehydrogenase (G6PDH) can be produced from natural G6PDH by reaction with the succinimide ester of biotin. Moreover, it is also possible to use a biotinyl enzyme prepared by using c-DNA.

It is sufficient for the purposes of this invention for the biotinyl enzyme to be allowed to make contact with avidin or streptoavidin which has been contacted with the biotin to be measured. Thus, the enzyme may be added to the biotin-containing solution prior to the reaction of the avidin or streptoavidin with the biotin to be measured, or it may also be added after the reactions. The suitable pH is usually at about 4 to 8.5, and the suitable temperature is usually from 20 to 45°C. The contacting time may be, for example, from 10-60 minutes at about 37°C.

After these contactings, the amount of the biotinyl enzyme which does not react with the avidin or streptoavidin is measured. The measurement is carried out according to a known method. For example, in the case of propionyl CoA carboxylase, ADP produced during the reaction is allowed to react in the presence of pyruvate kinase and lactate dehydrogenase, and the decrease in the NADH is colorimetrically determined. In the case of methylmalonyl CoA transcarboxylase, the oxalacetic acid produced is converted to malic acid in the presence of malate dehydrogenase, and the decrease of NADH is measured. Alternatively to the foregoing when propionyl CoA carboxylase is used, ATP produced by a reverse reaction is allowed to react in the presence of luciferase and luciferin, and thereby bioluminescence is produced. When this bioluminescence is measured by using a photon counter, An ATP concentration lower than about $10^{-14}$ M can be determined. This method is suitable for determining an extremely small amount of biotin.

When following the method of the invention, biotin is determined in a high sensitivity. Furthermore, the operation of the present method is a simple one.

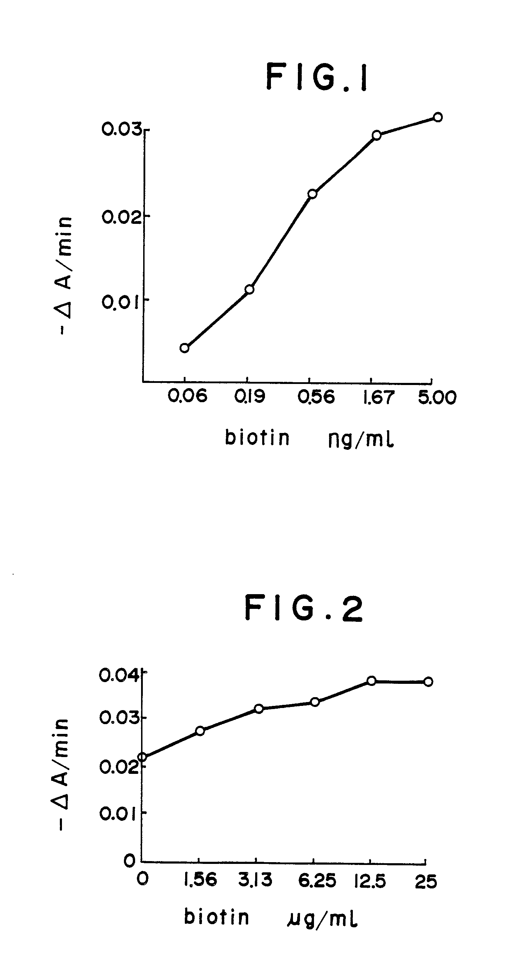

The following Examples illustrate the invention. In the Examples reference will be made to the accompanying drawing, wherein Figures 1 and 2 indicate the relation between the concentration of biotin and the decrease in rate of absorbance obtained in the Examples.

## EXAMPLES

## Example 1

50 µl of an aqueous sclution containing 10 µg of propionyl CoA carboxylase being a biotinyl enzyme were added to 50 µl portions of aqueous biotin solution containing 0 to 5 µg/ml of biotin, and 50 µl of aqueous avidin solution containing 15 µg of avidin was further added. The mixture was allowed to stand at room temperature for 20 minutes. Then 1 ml of a substrate solution of pH 8.0 containing 4 mM $MgCl_2$, 2 mM glutathione in reduced form, 2 mM ATP, 0.1 M KCl, 50 mM $NaHCO_3$, 1 mM phosphoenolpyruvic acid, 2.5 U/ml pyruvate kinase, 3.5 U/ml lactate dehydrogenase, 0.15 mM NADH and 2mM propiony CoA was added, and the absorbance at 340 nm at 30°C was measured at intervals of 30 seconds to determine the decrease rate of the absorbance. The results are indicated in Figure 1.

## Example 2

50 µl of an aqueous solution containing 0.14 U/ml of propionyl CoA carboxylase derived from NIH 1628 strain was added to 50 µl portions of aqueous biotin solution containing 0 to 25 ng/ml of biotin, and 50 µl of aqueous avidin solution containing 10 µg/ml of avidin was further added. The mixture was allowed to stand at 37°C for 20 minutes. Then, 1 ml of a substrate solution of pH 7.2 containing 0.16 mM propionyl CoA, 3 U/ml pyruvate kinase, 3.5 U/ml lactate dehydrogenase, 1.1 mM ATP, 6 mM $NaHCO_3$, 0.15 mM NADH, 1.3 mM PEP, 7.5 mM $MgCl_2$ and 50 mM Tris-HCl was added, and the decrease rate of the absorbance at 340 nm at 37°C was measured. The results are indicated in Figure 2. The sensitivity of the present assay was in 100 pg/ml to 12 ng/ml.

**Claims**

1. A method of measuring biotin comprising allowing there to co-exist in as aqueous medium biotin, avidin or streptoavidin and a biotinyl enzyme, and measuring the residual biotinyl enzyme activity as a measure of the biotin originally present.

2. A method as claimed in claim 1, wherein avidin or streptoavidin is contacted with the biotin to be measured and a biotinyl enzyme at the same time.

3. A method as claimed in claim 1 or 2, wherein the biotinyl enzyme is selected from propionyl CoA carboxylase, acetyl CoA carboxylase, pyruvate carboxylase, methylmalonyl CoA carboxylase, methylmalonyl CoA-transcarboxylase and methylcrotonyl CoA carboxylase.

4. A method as claimed in claim 1 or 2, wherein the biotinyl enzyme is a biotin-modified enzyme prepared by reacting a non-biotinyl enzyme with activated biotin.

5. A method as claimed in claim 4, wherein the biotinyl enzyme is biotin-induced glucose-6-phosphate dehydrogenase.

6. A method as claimed in any preceding claim, wherein, in measuring the biotinyl enzyme activity, a reaction is carried out which produces photoluminescence which is measured quantitatively using a photon counter.

7. A method as claimed in any one of claims 1 to 5, wherein ADP is produced and is allowed to react in the presence of pyruvate kinase and lactate dehydrogenase and the decrease in NADH thereby obtained is colorimetrically determined as a measure of the biotin originally present.

# F I G . 1

# F I G . 2